# DEMANDE DE BREVET EUROPEEN

(11) **EP 2 363 096 A1**
(43) Date de publication de la demande: **07.09.2011**
(21) Numéro de dépôt: 11156634.5
(22) Date de dépôt: 02.03.2011
(51) Int. Cl.: A61F 2/08

(54) **Bande de restauration de l'attache à un os d'un tissu mou tel qu'un tendon ou un ligament**

(30) Priorité: 07.05.2010 FR 1053601; 02.03.2010 US 309799 P
(71) Demandeur: Tornier, Inc., Edina, MN 55435 (US)
(72) Inventeur: Anderson, Justin C., Minnetonka, MN 55345 (US); Rushdy, Djamal, Eden Prairie, MN 55347 (US); Ohasi, Kevin L., Jamaica Plain, MA 02130 (US); Mattern, Ralph, San Diego, CA 92129 (US); Peterson, Dale, La Jolla, CA 92037 (US)
(74) Mandataire: Grand, Guillaume

(57) **Abrégé**

Cette bande (1) comprend un corps textile souple allongé (2) incluant, suivant sa direction longitudinale, des première (2A) et seconde (2B) parties longitudinales distinctes. Afin de retenir de manière fiable et pérenne un tissu mou (T) vis-à-vis d'un os (B), la première partie longitudinale (2A) est solidaire d'un moyen de fixation (10) adapté pour, après avoir passé au moins une portion de la seconde partie longitudinale (2B) au moins à travers le tissu mou (T) et l'avoir mise en prise avec l'os (B), le cas échéant en l'ayant fait passer dans un tunnel traversant (B₁) réalisé dans l'os, coopérer mécaniquement avec cette seconde partie longitudinale de manière à maintenir le corps textile (2) soumis à un effort de tension sous forme d'une boucle enserrant une partie du tissu mou, ainsi qu'une partie de l'os le cas échéant, afin de retenir le tissu mou vis-à-vis de l'os.

## Description

La présente invention concerne une bande de restauration de l'attache à un os d'un tissu mou, tel qu'un tendon ou un ligament. Elle concerne également une méthode de restauration correspondante.

Lorsqu'un tissu mou d'un être humain, tel qu'un tendon ou un ligament, est endommagé au niveau de son insertion d'attache à un os, il est connu de restaurer cette attache par une ou plusieurs bandes textiles, respectivement solidarisées à, à la fois, le tissu mou et l'os, et ce par tout moyen approprié, tel que des sutures et des vis. L'intervention chirurgicale correspondante est souvent longue et délicate à mettre en oeuvre. En outre, malgré le soin apporté par le chirurgien dans la réalisation de l'intervention, les risques de désolidarisation de la bande textile, tant au niveau du tissu mou que de l'os, sont élevés car, très souvent, les moyens de solidarisation utilisés fragilisent le tissu mou et/ou l'os. Ce constat est d'autant plus critique lorsqu'on cherche à restaurer de cette façon l'attache des tendons et ligaments de la coiffe des rotateurs à un os de l'épaule d'un être humain, en raison des fortes intensités de contrainte mécanique qui y sont atteintes lors des mouvements de l'épaule.

Dans un autre contexte, à savoir celui de la réparation ligamentaire du genou, JP-A-2002/058676 propose d'attraper un ligament de genou en forme de boucle, en passant à l'intérieur un élément filaire de suspension qu'il est possible à la fois de fermer sur lui-même, tout en ajustant sa longueur, par nouage ou par un moyen rapporté ad hoc, et de fixer à un os du genou. Cet élément filaire de suspension s'apparente donc à, en quelque sorte, une rallonge ajustable du tendon à réparer.

Le but de la présente invention est de proposer une bande et une méthode de restauration, qui permettent de retenir un tissu mou vis-à-vis d'un os de manière fiable et pérenne.

A cet effet, l'invention a pour objet une bande de restauration de l'attache à un os d'un tissu mou, telle que définie à la revendication 1. Cette bande permet de mettre en oeuvre une méthode de restauration de l'attache à un os d'un tissu mou, tel qu'un tendon ou un ligament, dans laquelle on dispose d'un corps textile souple allongé incluant, suivant sa direction longitudinale, des première et seconde parties longitudinales, la première partie longitudinale étant solidaire d'un moyen de fixation à la seconde partie longitudinale, et dans laquelle, après avoir fait passer au moins une portion de la seconde partie longitudinale au moins à travers le tissu mou et l'avoir mise en prise avec l'os, le cas échéant en l'ayant fait passer dans un tunnel traversant réalisé dans l'os, on fait coopérer mécaniquement le moyen de fixation avec cette seconde partie longitudinale de manière à maintenir le corps souple, que l'on a soumis à un effort de tension, sous forme d'une boucle enserrant une partie du tissu mou, ainsi qu'une partie de l'os le cas échéant, afin de retenir le tissu mou vis-à-vis de l'os.

Une des idées à la base de l'invention est de lier la bande textile au tissu mou en conformant le corps souple de cette bande en une boucle enfilée à travers le tissu mou et fermée sur elle-même par un moyen de fixation qui retient le corps souple sous tension. De cette façon, la solidarisation entre le corps de la bande et le tissu mou est réalisée essentiellement, voire exclusivement grâce à cet agencement en boucle, qui enserre une partie du tissu mou et la maintient vis-à-vis de l'os, en supportant des contraintes de tension élevées et en transmettant efficacement ces contraintes au tissu mou. De cette façon, des moyens de solidarisation rapportés au cours de l'intervention chirurgicale de mise en place de cette bande, tels que des sutures, ne sont pas nécessaires. D'ailleurs, comme expliqué plus en détail par la suite, la boucle précitée peut même être avantageusement prévue pour passer dans un tunnel traversant réalisé dans l'os : dans ce cas, aucun moyen de solidarisation rapporté au cours de l'intervention chirurgicale, telle que des vis, n'est nécessaire pour lier la bande textile à l'os, car cette dernière enserre alors une partie de l'os et lui transmet efficacement des contraintes de tension appliquées à la bande.

En pratique, le moyen de fixation selon l'invention, permettant de maintenir la boucle formée par le corps souple sous tension, peut prendre des formes de réalisation diverses, comme présenté en détail dans la suite, étant entendu que ces diverses formes induisent des variantes de mise en oeuvre pour la méthode chirurgicale de mise en place de la bande. Dans tous les cas, les performances de restauration sont remarquables.

De même, le corps souple de la bande selon l'invention peut être réalisé en divers matériaux textiles, au sens large du terme, permettant en particulier sa colonisation tissulaire. D'ailleurs, notamment grâce à la qualité d'attache de la bande au tissu mou et à l'os, le corps souple de la bande peut avantageusement être prévu, moyennant le ou les matériaux qui le constituent et la structure qu'il présente, aussi résistant et raide que le tissu mou à restaurer, voire davantage.

Des caractéristiques additionnelles avantageuses de la bande de restauration conforme à l'invention, prises isolément ou selon toutes les combinaisons techniquement possibles, sont spécifiées aux revendications dépendantes 2 à 15.

La bande selon l'invention permet également de mettre en oeuvre une méthode pour attacher un tissu mou à un os, comprenant des étapes selon lesquelles :
- on passe un corps souple allongé textile à travers le tissu mou, ce corps comprenant un moyen mécanique de fixation,
- on relie le corps textile à lui-même à l'aide du moyen mécanique de fixation, pour former une boucle,
- on met en prise le corps textile avec l'os, et
- on sert la boucle de manière à mettre sous tension le corps textile et enserrer une partie du tissu mou pour la retenir vis-à-vis du corps.

Suivant des caractéristiques additionnelles avantageuses de cette méthode :
- le corps textile présente une contrainte à la rupture supérieure à celle du tissu mou ;
- pour mettre en prise le corps textile avec l'os, on passe ce corps à travers un tunnel osseux réalisé dans l'os ;
- pour passer le corps textile à travers le tunnel osseux, on passe ce corps à travers ce tunnel osseux avant que la boucle ne soit formée ;
- pour passer le corps textile à travers le tissu mou, on passe le corps textile à travers le tissu mou une première fois, la méthode comprenant une étape supplémentaire consistant à passer le corps textile à travers le tissu mou une seconde fois ;
- pour passer le corps textile à travers le tissu mou, on passe le corps textile à travers le tissu mou en un premier point, le tunnel osseux étant un premier tunnel osseux, tandis que la méthode comporte l'étape supplémentaire selon laquelle le corps textile est passé à travers le tissu mou en un second point et on passe le corps textile à travers un second tunnel osseux réalisé dans l'os ;
- le passage du corps textile à travers le tissu mou au niveau des premier et second points et à travers les premier et second tunnels osseux intervient avant que le corps textile ne soit relié à lui-même par le moyen mécanique de fixation ;
- le corps textile est un premier corps textile, le tunnel osseux étant un premier tunnel osseux, la méthode comprenant l'étape supplémentaire selon laquelle un second corps textile est passé à travers le tissu mou et à travers un second tunnel osseux.

L'invention sera mieux comprise à la lecture de la description qui va suivre, donnée uniquement à titre d'exemple et faite en se référant aux dessins sur lesquels :
- la figure 1 est une vue schématique en élévation d'une bande de restauration selon un premier mode de réalisation conforme à l'invention ;
- la figure 2 est une vue en élévation, selon la flèche II de la figure 1, d'une zone partielle de cette bande de restauration ;
- la figure 3 est une coupe selon la ligne III-III de la figure 2, montrant uniquement une pièce appartenant à la bande de restauration des figures 1 et 2 ;
- la figure 4 est une vue en élévation selon la flèche IV de la figure 3 ;
- la figure 5 est une vue schématique illustrant l'utilisation de la bande de restauration de la figure 1 ;
- la figure 6 est une vue schématique en élévation selon la flèche VI de la figure 5 ;
- la figure 7 est une vue analogue à la figure 6, illustrant une variante d'utilisation de la bande de restauration ;
- les figures 8 et 9 sont des vues analogues à la figure 2, illustrant des variantes de réalisation d'une bande de restauration conforme à l'invention ;
- les figures 10 et 11 sont des vues schématiques en perspective, illustrant respectivement d'autres variantes de réalisation d'une bande de restauration conforme à l'invention ;
- la figure 12 est une vue analogue à la figure 1, illustrant une bande de restauration selon un second mode de réalisation conforme à l'invention ; et
- les figures 13 et 14 sont des vues schématiques en élévation, illustrant l'utilisation de la bande de restauration de la figure 12.

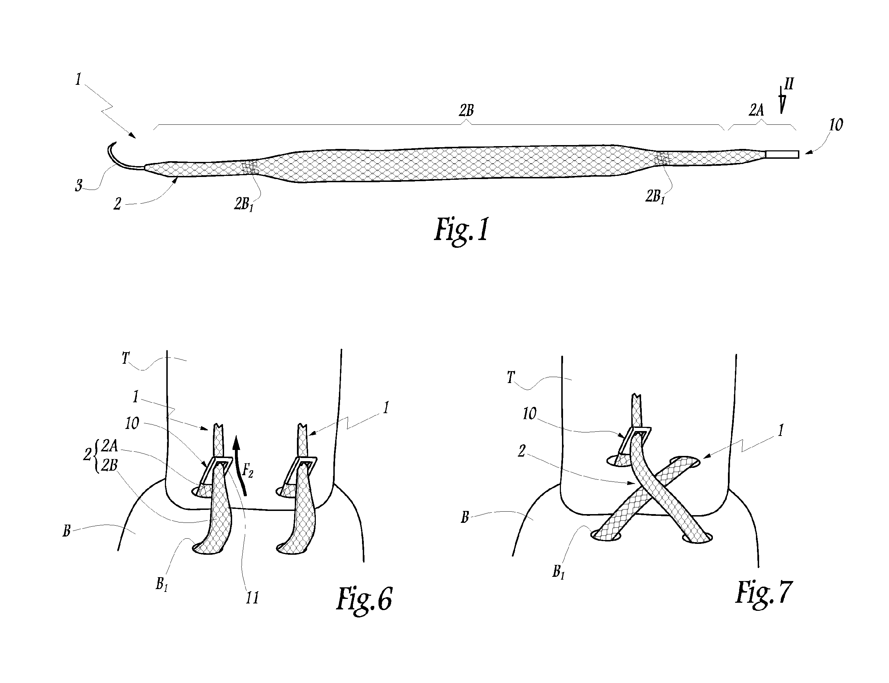

Sur la figure 1 est représentée une bande 1 destinée à restaurer l'attache d'un tissu mou, tel qu'un tendon ou un ligament, à un os.

Cette bande 1 comporte principalement un corps textile souple 2 allongé. A titre d'exemple préféré, ce corps 2 est une bandelette textile, composée notamment de fils tressés selon deux axes différents. A titre d'alternative, les fils de cette bandelette textile définissent trois axes différents. Chacun des fils de la bandelette est soit un monofilament, soit un multifilament, le cas échéant rectiligne ou tordu de manière hélicoïdale autour de l'axe central du fil. En pratique, les fils de cette bandelette textile sont réalisés soit en un matériau résorbable, auquel cas des exemples de matériaux sont l'hydroxybutyrate, tel que le Tephaflex (marque déposée), l'Artelon (marque déposée) et des polymères à base glycolique ou lactique, soit un matériau non résorbable, auquel cas des exemples de matériaux sont le polyester, le nylon, le polyétheréthercétone (PEEK), la soie, le polyacétale, le polyuréthane, le polyaramide, etc.

Dans tous les cas, le corps textile 2 de la bande 1 présente avantageusement une porosité favorisant la colonisation tissulaire.

Suivant un mode de réalisation préféré, le corps textile 2 présente des caractéristiques mécaniques remarquables, c'est-à-dire qu'il est avantageusement prévu pour être aussi résistant et raide que le tissu mou à restaurer par la bande 1. En particulier, dans le cas où la bande 1 est destinée à être utilisée au niveau de l'épaule d'un être humain, pour restaurer l'attache, à un os de l'épaule, d'un des tendons et ligaments de la coiffe des rotateurs, notamment le tendon sus-épineux, le corps textile 2 présente avantageusement :
- une contrainte à la rupture supérieure à 300 N, voire 450 N, et
- une raideur comprise entre 15 et 300 N/mm.

Plus généralement, le corps textile 2 est prévu pour présenter une grande contrainte à la rupture, pour des raisons évidentes de résistance mécanique et de tenue aux contraintes, ainsi que pour présenter une raideur proche de celle du tissu mou à restaurer, notamment comprise entre 0,2 et 2 fois, de préférence 0,5 et 1 fois environ, la raideur du tissu à restaurer : de cette façon, la colonisation tissulaire du corps textile 2 est favorisée.

Comme indiqué schématiquement sur la figure 1, le corps 2 de la bande 1 est constitué de deux parties longitudinales 2A et 2B successives suivant la direction longitudinale du corps : dans l'exemple de réalisation considéré, la partie 2A correspond à l'une des deux zones terminales opposées du corps 2, incluant l'extrémité libre de cette zone terminale, qui, comme expliqué en détail plus loin, est solidairement pourvue d'une pièce 10 rigide ou semi-rigide. La partie 2B correspond au reste du corps 2, c'est-à-dire, dans l'exemple de réalisation considérée ici, à la zone courante du corps 2 et la zone terminale de ce corps, opposée à la partie 2A.

Avantageusement, comme dans l'exemple représenté à la figure 1, la partie 2B du corps 2 présente, à proximité de chacune de ses extrémités longitudinales, une région de consolidation 2B₁ au niveau de laquelle la section transversale du corps 2 change, notamment par écrasement sur lui-même du matériau constituant le corps : ainsi, entre les deux régions de consolidation 2B₁ suivant la direction longitudinale du corps 2, ce dernier présente une section transversale plus importante que celle de part et d'autre de ces régions de consolidation. Cette disposition facilite les manipulations des zones terminales du corps 2, comme on le comprendre mieux par la suite, tandis que, eu égard à sa plus grande section transversale, l'effet de restauration par la zone courante du corps 2 est renforcé.

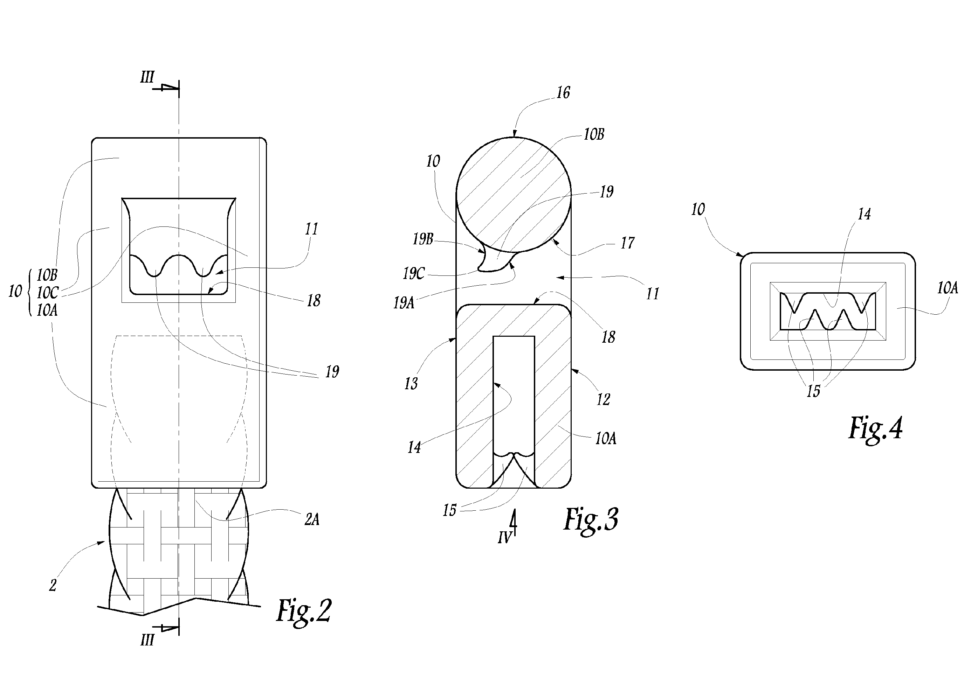

La pièce 10 prévue à l'extrémité libre de la partie 2A du corps 10 est montrée plus en détail sur les figures 2 à 4. Cette pièce 10 se présente, dans l'exemple de réalisation considéré sur ces figures, sous la forme globale d'un parallélépipède dont la dimension la plus grande, que l'on peut qualifier de longueur, s'étend suivant la direction longitudinale du corps 2. Suivant cette direction longitudinale, la pièce 10 comporte deux parties terminales opposées 10A et 10B, qui sont reliées l'une à l'autre par deux parois latérales opposées 10C appartenant à la pièce 10, et qui délimitent entre elles, conjointement avec les parois latérales 10C, un trou traversant 11. Le trou 11 s'étend transversalement à la direction longitudinale précitée, en reliant l'une à l'autre les deux faces principales opposées 12 et 13 de la pièce 10.

La partie terminale 10A de la pièce 10, qui est tournée vers le corps 2, délimite intérieurement une cavité borgne 14 de réception et de solidarisation à la partie 2A du corps 2. Cette cavité borgne 14 est dimensionnée de façon complémentaire à l'extrémité libre de la partie de corps 2A, en débouchant sur le chant d'extrémité longitudinale de la partie de pièce 10A, permettant ainsi d'introduire et de loger la partie de corps 2A dans la cavité 14. Afin d'immobiliser la portion de la partie de corps 2A, introduite dans la cavité 14, cette dernière est avantageusement pourvue de pointes anti-retour 15, bien visibles sur les figures 3 et 4 : chacune de ces pointes 15, conformée à la façon d'un aileron de requin, présente, en direction du débouché de la cavité 14, une face de rampe, inclinée vers l'intérieur de la cavité 14, en étant potentiellement légèrement bombée, tandis que, du côté opposé, la pointe 15 délimite un épaulement, potentiellement légèrement creusé. De cette façon, lors de l'introduction de l'extrémité libre de la partie de corps 2A dans la cavité 14, cette extrémité glisse facilement contre la surface de rampe des pointes 15, jusqu'à franchir le sommet de ces pointes, en direction du fond de la cavité 14. Le dégagement ultérieur de la partie de corps 2A est empêché par l'épaulement des pointes 15, qui bloquent mécaniquement la partie de corps 2A suivant une direction opposée à celle d'introduction de la partie de corps 2A dans la cavité 14.

Avantageusement, comme bien visible sur la figure 4, les pointes anti-retour 15 sont réparties régulièrement suivant la plus grande dimension de la section transversale de la cavité 14, en s'étendant, de manière alternée, depuis l'une et l'autre des deux faces principales opposées de la cavité 14.

La partie terminale 10B de la pièce 10 présente, sur son côté opposé à la partie terminale 10A, une surface extérieure 16 incurvée ou, plus généralement, convexe, notamment afin de limiter les effets traumatiques de la pièce 10 après implantation de la bande 1.

Dans l'exemple de réalisation considéré sur les figures 2 à 4, la partie terminale 10B se présente, pour l'essentiel, sous forme d'une barre cylindrique à section circulaire, centrée sur un axe perpendiculaire à la direction longitudinale de la pièce 10, la surface incurvée précitée 16 correspondant à une portion de la surface de cette barre cylindrique. La portion 17 de la surface extérieure de cette barre cylindrique, diamétralement opposée à la surface incurvée 16, délimite en partie le trou traversant 11, comme bien visible sur la figure 3. En regard de cette barre cylindrique, le trou 11 est délimité par une surface plane 18 formée par la partie terminale 10A.

Avantageusement, le trou d'enfilage 11 est pourvu intérieurement de pointes anti-retour 19, bien visibles sur les figures 2 et 3. Dans l'exemple de réalisation considéré sur les figures, ces pointes anti-retour 19 s'étendent en saillie depuis la surface 17 de la partie terminale 10A, en étant conformées en aileron de requin : du côté dirigé vers la face principale 12 de la pièce 10, chacune de ces pointes 19 délimite une surface de rampe 19A, avantageusement bombée, tandis que du côté de la face principale opposée 13, la pointe 19 délimite un épaulement 19B, avantageusement creusé. De cette façon, lorsqu'un élément souple est enfilé dans le trou 11, depuis la face 12 de la pièce 10, en direction de la face 13, cet élément souple glisse contre la surface de rampe 19A des pointes 19, jusqu'à franchir le sommet 19C de ces pointes, tandis que, dans le sens opposé à celui de cet enfilage, l'élément souple est bloqué mécaniquement par l'épaulement 19B des pointes 19, en étant accroché par les sommets 19C. Ainsi, les pointes anti-retour 19 retiennent l'élément souple précité dans le sens opposé à celui d'enfilage de cet élément souple dans le trou 11.

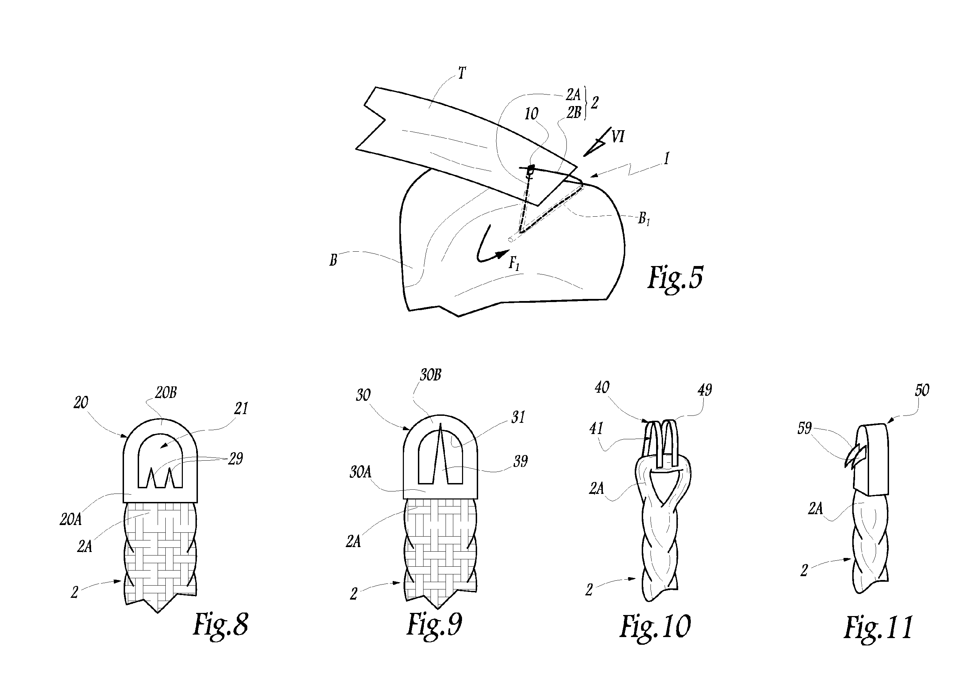

L'utilisation de la bande 1 va maintenant être décrite, en particulier en regard des figures 5 et 6, en vue de restaurer l'attache d'un tissu mou T à un os B. A titre d'exemple, l'os B est un humérus d'un être humain tandis que le tissu mou T est l'un des tendons ou ligaments appartenant à la coiffe des rotateurs, associée à l'humérus précité.

Préalablement à la mise en place proprement dite de la bande 1, on réalise un tunnel B₁ à travers l'os B, dans la zone de ce dernier où il est prévu d'attacher le tissu mou T. Ainsi, comme indiqué schématiquement à la figure 5, ce tunnel osseux B₁ peut être prévu coudé, de manière à déboucher, à chacune de ses deux extrémités, sur sensiblement la même face de l'os B. Pour ce faire, un forêt ou un ancillaire chirurgical analogue est introduit dans la face précitée de l'os B, et ce à deux reprises, de manière à réaliser deux parties de tunnel qui se rejoignent dans l'épaisseur de l'os B. Bien entendu, en variante non représentée, un tunnel osseux B1 rectiligne peut être réalisé à travers l'os B, moyennant le fait que ce tunnel débouche alors sur deux faces différentes de l'os B. A l'inverse, on peut envisager de réaliser des tunnels osseux plus élaborés que le tunnel B₁ montré sur la figure 5, l'essentiel étant que le tunnel osseux traverse l'os B dans une zone résistante et stable de celui-ci.

Après avoir réalisé le tunnel osseux B₁ on enfile l'extrémité libre de la partie 2B du corps 2 de la bande 1 à l'intérieur de ce tunnel, après avoir fait passer cette extrémité libre de la partie de corps 2B à travers le tissu mou T. En pratique, pour faciliter le passage traversant de la partie de corps 2B à travers le tissu mou T, ainsi que le guidage de cette partie de corps à l'intérieur du tunnel osseux B₁ l'extrémité libre de la partie de corps 2B est avantageusement munie d'une aiguille rapportée 3, comme représenté sur la figure 1, ou d'un élément similaire. Dans tous les cas, on comprend que la faible section transversale de la zone terminale de la partie de corps 2B facilite l'enfilage de cette partie de corps successivement à travers le tissu mou T et dans le tunnel osseux B₁.

L'enfilage de l'extrémité libre de la partie de corps 2B se poursuit dans le tunnel osseux B₁, jusqu'à faire ressortir cette extrémité libre par le débouché du tunnel, autre par lequel l'extrémité libre a été introduite, comme indiqué par la flèche coudée F₁ sur la figure 5. Comme représenté sur la figure 6, l'extrémité libre de la partie de corps 2B émerge ainsi de l'os B. Cette extrémité libre est alors enfilée dans le trou 11 de la pièce 10, en traversant ainsi cette dernière de sa face 12 à sa face 13, comme indiqué par la flèche F₂ sur la figure 6.

Avantageusement, l'aiguille 3 facilite cet enfilage dans le trou 11.

Le corps 2 de la bande 1 forme ainsi une boucle, qui est fermée sur elle-même au niveau de la pièce 10 et à l'intérieur de laquelle sont agencées une partie du tissu mou T et une partie de l'os B, comme montré schématiquement à la figure 5.

L'enfilage de la partie de corps 2B à travers le trou 11 se poursuit de manière à serrer progressivement la boucle précitée autour du tissu mou T et de l'os B : le corps 2 se trouve ainsi progressivement tendu suivant sa direction longitudinale, en enserrant le tissu mou T et l'os B. Grâce aux pointes anti-retour 19, l'enfilage de la partie de corps 2B est unidirectionnelle de sorte que, au fur et à mesure que le corps 2 est tendu, la boucle formée par ce corps se resserre, sans desserrement possible.

On comprend que, au fur et à mesure du resserrement de la boucle formée par le corps 2, la portion longitudinale de la partie de corps 2B non encore introduite à travers le tissu mou T et l'os B se réduit progressivement, tandis que la portion qui émerge de la pièce 10, après avoir été enfilée à travers son trou 11, augmente, jusqu'à application d'un effort de tension considéré comme suffisant par le chirurgien. L'excédent du corps 2, émergeant de la pièce d'enfilage 10, peut alors être sectionné et retiré.

Ainsi, la bande 1 retient sous tension le tissu mou T vis-à-vis de l'os B, de manière ferme et stable. Grâce au fait que la bande 1 est passée à travers le tissu mou T, puis conformée en une boucle enserrant une partie de ce tissu mou, la liaison entre la bande 1 et le tissu mou T est pérenne et supporte des contraintes de tension élevées, sans nécessiter l'adjonction de moyen de fixation additionnel entre le corps 2 et le tissu mou, ces contraintes étant efficacement transmises du corps textile 2 au tissu mou T. De même, grâce au fait que la bande 1 est passée à travers une partie de l'os B, en formant une boucle enserrant cette partie d'os, elle se trouve liée fermement à l'os B, sans nécessiter l'adjonction de moyen de fixation additionnel entre le corps 2 et l'os.

On comprend que l'enserrement du tissu mou T par le corps textile 2 favorise la rapide et complète colonisation tissulaire de ce dernier lorsqu'il présente une porosité ad hoc, étant entendu que le corps textile 2 est prévu pour maintenir cette porosité lorsqu'il est tendu et conformé en boucle. Via cette colonisation, la transmission des efforts entre le corps 2 et le tissu mou T est encore améliorée, avec une répartition qui peut évoluer dans le temps, au fur et à mesure de la cicatrisation du tissu mou.

En pratique, comme représenté sur la figure 6, plusieurs bandes 1 peuvent être utilisées, de manière juxtaposée, pour renforcer la restauration de l'attache entre le tissu mou T et l'os B.

A titre de variante illustrée par la figure 7, la même bande 1 peut être utilisée pour réaliser deux boucles croisées, moyennant une longueur suffisante pour le corps 2 de cette bande.

Diverses formes de réalisation peuvent être envisagées pour la pièce d'enfilage 10, comme considéré sur les figures 8, 9 et 10.

Ainsi, à la figure 8, la bande 1 est pourvue solidairement, en remplacement de la pièce d'enfilage 10, d'une pièce d'enfilage 20 dont la fonction est similaire à celle de la pièce 10 en vue de restaurer l'attache d'un tissu mou à un os. Par comparaison à la pièce d'enfilage 10, la pièce d'enfilage 20 délimite un trou traversant 21 d'enfilage, fonctionnellement similaire au trou d'enfilage 11, et agencé, suivant la direction longitudinale de la bande 1, entre deux parties terminales 20A et 20B de la pièce 20. Par comparaison à la partie 10A de la pièce 10, la partie 20A de la pièce 20 présente une dimension longitudinale plus petite, liée notamment au fait que la solidarisation de la pièce 20 au corps 2 de la bande 1 est réalisée de manière différente de celle de la pièce 10 : plutôt que de prévoir que l'extrémité libre de la partie de corps 2A est introduite et retenue dans une cavité borgne similaire à la cavité 14, on envisage que l'extrémité libre de la partie de corps 2A est collée, cousue, entremêlée ou, plus généralement reliée solidairement par tout moyen approprié à la partie terminale 20A de la pièce 20.

Par ailleurs, indépendamment de l'aspect qui précède, la partie terminale 20B de la pièce 20 se présente sous la forme d'un arceau, autrement dit sous une forme géométrique différente de la barre cylindrique constituée par la partie terminale 10B de la pièce 10. Ainsi, cela illustre la multitude de conformations envisageables pour cette partie terminale de la pièce d'enfilage.

De plus, également de manière indépendante à ce qui précède, le trou 21 de la pièce 20 est pourvu de pointes anti-retour 29, fonctionnellement similaires aux pointes 19 de la pièce 10, à la différence que les pointes 29 s'étendent en saillie de la partie terminale 20A alors que les pointes 19 sont prévues saillantes depuis la partie terminale 10B de la pièce 10. De nouveau, cela illustre les diverses possibilités d'agencement positionnel relatif des pointes anti-retour dans le trou d'enfilage délimité par la pièce d'enfilage. Ainsi, à titre de variante non représentée, le trou 21 de la pièce 20 est, en plus des pointes 29, muni de pointes identiques aux pointes 19.

A la figure 9, le corps 2 de la bande 1 est solidairement pourvu, en remplacement de la pièce d'enfilage 10 ou de la pièce d'enfilage 20, d'une pièce d'engagé 30 définissant un trou d'enfilage 31 délimité entre des parties terminales 30A et 30B de la pièce 30, fonctionnellement similaires au trou 21 et aux parties 20A et 20B de la pièce 20. La pièce 30 se distingue de la pièce 20 par le remplacement des pointes anti-retour 29 par une languette 39, qui s'étend en saillie depuis la partie terminale 30A et dont l'extrémité libre opposée est prévue pour venir s'appuyer élastiquement contre l'arceau que constitue la partie terminale 30B. De cette façon, cette languette 39 s'écarte de la partie terminale 30B lors de l'enfilage de la partie 2B du corps 2, en particulier par déformation souple de sa zone de liaison à la partie terminale 30A, à la façon d'une charnière, tandis que, une fois que la boucle formée par le corps 2 est resserrée avec l'intensité souhaitée, l'extrémité libre de la languette 39 bloque la partie de corps 2B contre la partie terminale 30B, par effet de coincement.

A la figure 10, l'extrémité libre de la partie 2A du corps 2 est, en remplacement des pièces d'enfilage 10, 20 ou 30, pourvue d'une pièce d'enfilage 40 sous forme d'un anneau délimitant intérieurement un trou d'enfilage 41 fonctionnellement similaire au trou d'enfilage 11, 21 ou 31. L'anneau de la pièce 40 est associé à un second anneau 49 structurellement similaire à l'anneau de la pièce 40. La fonction de cet anneau 49 est de bloquer mécaniquement la partie 2B du corps 2 après que cette dernière a été enfilée dans le trou 41 et que la boucle formée par le corps 2 a été resserrée avec l'intensité désirée, et ce par coincement. Pour ce faire, un mode opératoire envisageable consiste à faire passer la partie de corps 2B à travers, à la fois, la pièce d'enfilage 40 et l'anneau 49, puis à la réintroduire à travers la pièce 40, en contournant par l'extérieur l'anneau 49.

A la figure 11 est représentée une autre variante d'une pièce 50 prévue solidaire de l'extrémité libre de la partie 2A du corps 2 et conçue pour se fixer, par accrochage, à la partie 2B du corps 2 après que cette dernière a été passée à travers le tissu mou T et dans un tunnel réalisé à travers l'os B. Cette pièce d'accrochage 50 est pourvue de crochets 59 adaptés pour s'ancrer, par pénétration, dans le matériau constituant la partie 2B du corps 2 et ainsi fixer la partie de corps 2A pour que le corps 2 forme une boucle serrée avec une intensité désirée. On comprend que les crochets 59 maintiennent fermement un engagement mécanique avec la partie de corps 2B tant que le corps 2 est soumis à un effort longitudinal de tension.

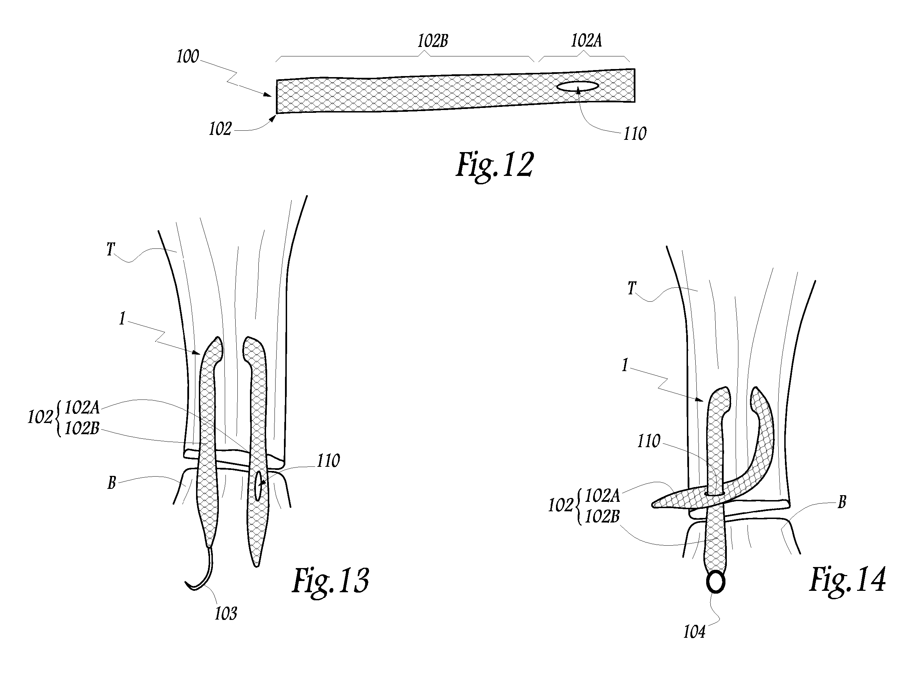

Sur les figures 12 à 14 est considérée une bande 100 destinée à restaurer l'attache d'un tissu mou T, tel qu'un tendon ou un ligament, à un os B. Cette bande 100 comporte un corps souple allongé 102 qui est structurellement et fonctionnellement similaire au corps 2 de la bande 1.

La bande 100 se distingue de la bande 1 par la forme de réalisation de son moyen permettant de fixer l'une à l'autre des parties longitudinales distinctes 102A et 102B du corps 102, de manière que ce corps 102 forme une boucle fermée sur elle-même. Plus précisément, la partie de corps 102A délimite, dans sa portion courante, une fente 110 dimensionnée pour permettre d'y enfiler la partie de corps 2B, en y introduisant d'abord l'extrémité libre de cette partie de corps 2B, puis en resserrant la boucle alors formée par le corps 102.

En pratique, la fente 110 peut présenter une forme extrêmement simple, consistant en une découpe longitudinale du matériau constituant la partie du corps 2A. A titre de variante non représentée, le bord de cette fente 110 peut être renforcé par une pièce rigide ou semi-rigide formant un oeillet. A titre d'option avantageuse, cet oeillet est intérieurement pourvu d'un ou de plusieurs éléments de blocage mécanique, fonctionnellement, voire structurellement similaires aux pointes anti-retour 19 ou 29 ou à la languette 39 décrites plus haut.

Dans tous les cas, la fente 110 permet de fixer à la partie de corps 2A la partie de corps 2B enfilée dans cette fente 110, pour maintenir sous tension le corps 2 conformé ainsi en une boucle.

La bande 100 peut être utilisée de la même façon que la bande 1, comme décrit plus haut. A titre de variante, les figures 13 et 14 illustrent une autre possibilité d'utilisation de la bande 100, qui est d'ailleurs applicable à la bande 1 : plutôt que de faire passer la partie de corps 102B à travers, à la fois, le tissu mou T et l'os B, cette partie de corps 102B est passée uniquement à travers le tissu mou T, avantageusement à l'aide d'une aiguille 103 similaire à l'aiguille 3 pour la bande 1, puis, après avoir enfilé cette partie de corps 102B à travers la fente 110 de la partie de corps 102A et serrer la boucle ainsi formée par le corps 102 avec l'intensité désirée, la partie de corps 102B est reliée à l'os B par un élément de solidarisation rapporté 104, telle qu'une vis ou, plus généralement, un ancrage osseux, comme représenté sur la figure 14.

Divers aménagements et variantes aux bandes 1 et 100 décrites jusqu'ici, ainsi qu'à leurs procédés d'utilisation, sont envisageables. A titre d'exemples :
- l'agencement directionnel entre la partie de corps 2A ou 102A et les trous d'enfilage 11, 21, 31, 41 ou la fente d'enfilage 110 n'est pas limité à ce qui est représenté ;
- après la mise en place, proprement dite, de la bande 1 ou 100, cette bande peut être suturée au tissu mou T, en particulier pour éviter des petits débattements entre eux, qui, à la longue, pourraient cisailler le corps 2 ou 102 ; dans cette optique, le corps textile 2 de la bande est avantageusement conçu pour, en plus des performances mécaniques de contrainte à la rupture et de raideur évoquées précédemment, présenter une résistance d'arrachement des sutures supérieure à 150 N ;

- la mise en oeuvre chirurgicale peut être réalisée aussi bien en chirurgie ouverte, qu'en chirurgie endoscopique ;
- la partie de corps 2B ou 102B peut, au moins localement, être enduite d'un lubrifiant, être gainée ou être traitée en surface afin de faciliter son glissement à travers les trous d'enfilage 11, 21, 31 et 41 ou la fente d'enfilage 110, ainsi qu'à travers le tissu mou T et, le cas échéant, dans le tunnel B₁ réalisé à travers B ;
- le matériau constituant le corps 2 peut être conçu pour, en particulier après avoir fait passer le corps 2 à travers le tissu mou T et, le cas échéant, l'os B, présenter des éléments transversalement saillants, de manière à renforcer la coopération mécanique de blocage entre la partie de corps 2B ou 102B et les trous d'enfilage 11, 21, 31 et 41 ou la fente d'enfilage 110 ; et/ou
- comme illustré sur les figures 5, 6, 13 et 14, la bande 1 ou 101 peut être utilisée seule pour restaurer l'attache du tissu mou T à l'os B ; en variante, cette bande peut être utilisée conjointement à un patch que la bande vient maintenir contre le tissu mou T, notamment afin de couvrir une zone tissulaire plus étendue.

## Revendications

1. Bande (1 ; 101) de restauration de l'attache à un os (B) d'un tissu mou (T), tel qu'un tendon ou un ligament,
comprenant un corps textile souple allongé (2 ; 102) incluant, suivant sa direction longitudinale, des première (2A ; 102A) et seconde (2B ; 102B) parties longitudinales, dans laquelle la première partie longitudinale (2A ; 102A) est solidaire d'un moyen de fixation (10 ; 20 ; 30 ; 40 ; 50 ; 110) adapté pour, après avoir passé au moins une portion de la seconde partie longitudinale (2B ; 102B) au moins à travers le tissu mou (T) et l'avoir mise en prise avec l'os (B), le cas échéant en l'ayant fait passer dans un tunnel traversant (B₁) réalisé dans l'os, coopérer mécaniquement avec cette seconde partie longitudinale de manière à maintenir le corps textile (2 ; 102) soumis à un effort de tension sous forme d'une boucle enserrant une partie du tissu mou, ainsi qu'une partie de l'os le cas échéant, afin de retenir le tissu mou vis-à-vis de l'os.

2. Bande de restauration de l'attache, à un os d'une épaule, d'un des tendons et ligaments appartenant à la coiffe des rotateurs, bande qui est conforme à la revendication 1.

3. Bande suivant l'une des revendications 1 ou 2, **caractérisée en ce que** le corps textile (2 ; 102) présente une porosité de colonisation par le tissu mou (T), y compris lorsqu'il est soumis à l'effort de tension.

4. Bande suivant l'une quelconque des revendications précédentes, **caractérisée en ce que** le corps textile (2 ; 102) présente une contrainte à la rupture supérieure à 300 N, de préférence 450 N.

5. Bande suivant l'une quelconque des revendications précédentes, **caractérisée en ce que** le corps textile (2 ; 102) présente une raideur comprise entre 15 et 300 N/mm.

6. Bande suivant l'une quelconque des revendications précédentes, **caractérisée en ce que** le corps textile (2 ; 102) présente une résistance à l'arrachement de suture supérieure à 150 N.

7. Bande suivant l'une quelconque des revendications précédentes, **caractérisée en ce que** le corps textile (2 ; 102) est composé de fils tressés.

8. Bande suivant l'une quelconque des revendications précédentes, **caractérisée en ce que** le moyen de fixation (10 ; 20 ; 30 ; 40 ; 110) définit un trou d'enfilage (11 ; 21 ; 31 ; 41 ; 110) pour la seconde partie longitudinale (2B ; 102B).

9. Bande suivant la revendication 8, **caractérisée en ce que** le moyen de fixation (10 ; 20 ; 30 ; 40) comporte une pièce d'enfilage (10 ; 20 ; 30 ; 40) rigide ou semi-rigide, qui délimite au moins partiellement le trou d'enfilage (11 ; 21 ; 31 ; 41) et qui est rapportée solidairement à la première partie longitudinale (2A), en particulier à une extrémité libre de cette première partie longitudinale.

10. Bande suivant la revendication 8, **caractérisée en ce que** le trou d'enfilage consiste en une fente (110) ménagée à travers la matière constituant la première partie longitudinale (1 02A) du corps textile (102).

11. Bande suivant l'une des revendications 8 à 10, **caractérisée en ce que** le moyen de fixation (10 ; 20 ; 30 ; 40) est associé à au moins un élément de blocage mécanique (19 ; 29 ; 39 ; 49) adapté pour, dans le sens opposé à l'effort de tension, retenir la seconde partie longitudinale (2B ; 102B) enfilée dans le trou d'enfilage (11 ; 21 ; 31 ; 41).

12. Bande suivant la revendication 11, **caractérisée en ce que** l'élément de blocage (19 ; 29) est agencé à l'intérieur du trou d'enfilage (11 ; 21) et conçu pour s'accrocher à la seconde partie longitudinale (2B).

13. Bande suivant la revendication 11, **caractérisée en ce que** l'élément de blocage (39 ; 49) est conçu pour coincer la seconde partie longitudinale (2B) contre la pièce d'enfilage (30 ; 40).

14. Bande suivant l'une quelconque des revendications 1 à 7, **caractérisée en ce que** le moyen de fixation (50) comporte une pièce (50) d'accrochage à la seconde partie longitudinale (2B), cette pièce d'accrochage étant rapportée solidairement à la première partie longitudinale (2A), en particulier à une extrémité libre de cette première partie longitudinale.

15. Bande suivant l'une des revendications 9 ou 14, **caractérisée en ce que** la pièce d'enfilage (10) ou la pièce d'accrochage délimite une cavité borgne (14) de réception d'une extrémité libre de la première partie longitudinale (2A), cette cavité borgne étant pourvue de reliefs (15) de blocage mécanique de cette extrémité libre.
